# EUROPEAN PATENT APPLICATION

(11) **EP 4 299 052 A1**
(43) Date of publication of application: **03.01.2024**
(21) Application number: 22305964.3
(22) Date of filing: 30.06.2022
(51) Int. Cl.: A61K 9/00, A61K 47/12, A61K 47/14, A61K 9/14, A61K 9/48

(54) **SOLID COMPOSITIONS COMPRISING A PEPTIDE OR A PROTEIN AND A PERMEATION ENHANCER**

(71) Applicant: Adocia, 69003 Lyon (FR)
(72) Inventor: NAESSENS, Ulysse, 69250 Curis au Mont d'Or (FR); SOULA, Gérard, 69330 Meyzieu (FR)
(74) Representative: Casalonga

(57) **Abstract**

The invention relates to solid composition comprising a peptide or a protein and a permeation enhancer, abbreviated PE, chosen from the group consisting of NAC (8-N-(2-hydroxybenzoyl)aminocaprylate), NAD (10-N-(2-hydroxybenzoyl)aminodecanoic acid), 5-CNAC (8-N-(5-chlorosalicyloyl)aminocaprylic acid), 4-MOAC (8-N-(2-hydroxy-4-methoxybenzoyl)aminocaprylic acid), 4-CNAB (4-N-(2-hydroxy-4-chlorobenzoyl)aminobutanoic acid) and salts thereof, in particular the sodium salt of (8-N-(2- hydroxybenzoyl)aminocaprylate), also called SNAC, and their use in medicine.

## Description

The present invention concerns compositions comprising a peptide and a permeation enhancer, abbreviated PE, chosen from the group consisting of NAC (8-N-(2-hydroxybenzoyl)aminocaprylate), NAD (10-N-(2-hydroxybenzoyl)aminodecanoic acid), 5-CNAC (8-N-(5-chlorosalicyloyl)aminocaprylic acid), 4-MOAC (8-N-(2-hydroxy-4-methoxybenzoyl)aminocaprylic acid), 4-CNAB (4-N-(2-hydroxy-4-chlorobenzoyl)aminobutanoic acid) and salts thereof, in particular their sodium or potasium salt, and more particularly the sodium or the potassium salt of (8-N-(2-hydroxybenzoyl)aminocaprylate), respectively called SNAC or KNAC, and their use in medicine.

Current peptides or protein therapies rely mainly on the parenteral way of administration.

Although oral delivery is clearly a must have for the treatments, in particular in terms of comfort of administration and improved compliance to the treatment, the hurdles for obtaining an oral way of administration for peptides and proteins are numerous and very challenging.

Among these hurdles can be cited a low bioavailability, a great variability of the bioavailability, a difficult processability, a slow solubilization of the permeation enhancer and/or an absorption site which can be aggressive for the active principle.

The peptides or proteins which are marketed under oral form are mostly small cyclic non-acylated or non-pegylated peptides. By small is meant a molecular weight of less or equal to 1200 Da. There we can cite Cyclosporin, Octreotide and Desmopressin.

However when talking about peptides or proteins not falling into this class, we can only cite Rybelsus^{®} as marketed peptide product which is a long acting GLP-1 RA (semaglutide) combined with a permeation enhancer (SNAC) in order to allow the oral delivery.

Although this product is marketed it still has some drawbacks, such as a low bioavailability, a great variability of bioavailability between different administrations, a slow solubilization of the permeation enhancer and also a constraint regarding the dosage protocol (fasting at least 30 minutes after Rybelsus^{®} dosage).

The invention proposes a way to solve at least part of the above cited problems.

In particular the invention aims at:
- improving the bioavailability and/or
- having a very short window of absorption and/or
- decreasing the variability of bioavailability between administrations.

This last feature would in particular allow to use short acting APIs as a great variability of absorption for short acting APIs would lead to great variability of API concentration in the blood, and thus the risk that the patient has a too low or too large dose of API.

This is surprisingly that the applicant has found that a composition according to the invention solves at least one of the technical problems cited above.

The composition according to the invention relates to a solid composition comprising a peptide or a protein and a permeation enhancer, abbreviated PE, chosen from the group consisting of NAC (8-N-(2- hydroxybenzoyl)aminocaprylate), NAD (10-N-(2-hydroxybenzoyl)aminodecanoic acid), 5-CNAC (8-N-(5-chlorosalicyloyl)aminocaprylic acid), 4-MOAC (8-N-(2-hydroxy-4-methoxybenzoyl)aminocaprylic acid), 4-CNAB (4-N-(2-hydroxy-4-chlorobenzoyl)aminobutanoic acid) and salts thereof, in particular the sodium or the potassium salt of (8-N-(2-hydroxybenzoyl)aminocaprylate), respectively called SNAC or KNAC, and more particularly SNAC.

In an embodiment, the composition comprises at least 300 mg/g of PE relative to the total weight of the composition.

In an embodiment, the composition comprises at least 400 mg/g of PE relative to the total weight of the composition.

In an embodiment, the composition comprises at least 500 mg/g of PE relative to the total weight of the composition.

In an embodiment, the composition comprises at least 600 mg/g of of PE relative to the total weight of the composition.

In an embodiment, the composition comprises at least 700 mg/g of of PE relative to the total weight of the composition.

In an embodiment, the composition comprises at least 800 mg/g of of PE relative to the total weight of the composition.

In an embodiment, the composition comprises at least 900 mg/g of of PE relative to the total weight of the composition.

In an embodiment, the composition comprises at most 990 mg/g of of PE relative to the total weight of the composition.

In an embodiment, the composition comprises at most 980 mg/g of of PE relative to the total weight of the composition.

In an embodiment, the composition comprises at most 950 mg/g of of PE relative to the total weight of the composition.

In an embodiment, the composition comprises at most 900 mg/g of of PE relative to the total weight of the composition.

In an embodiment, the composition comprises at most 800 mg/g of of PE relative to the total weight of the composition.

The composition according to the invention relates to a solid composition comprising a peptide or a protein, a PE and a lubricant.

The composition according to the invention relates to a solid composition comprising a peptide or a protein, a PE and a pH modifier.

The invention also relates to a unitary solid dosage comprising or consisting of the composition of the invention.

In an embodiment, the unitary solid dosage comprises a peptide or a protein and at least 300 mg/g of PE.

The invention also relates to a unitary solid dosage comprising a peptide or a protein and at least 300 mg/g of PE, said unitary solid dosage comprising at least 50 mg of PE.

The invention also relates to a pharmaceutical formulation comprising a solid composition as disclosed in this specification.

The invention also relates to a solid composition for oral delivery comprising a peptide or a protein and a PE.

The invention also relates to a method of treatment comprising the step of orally taking a solid composition as disclosed in the specification for preventing or treating a disease.

According to an embodiment the composition is an oral composition.

By "peptides" is meant amides derived from two or more amino carboxylic acid molecules (the same or different) by formation of a covalent bond from the carbonyl carbon of one to the nitrogen atom of another with formal loss of water. The term is usually applied to structures formed from α-amino acids, but it includes those derived from any amino carboxylic acid. This definition comes from IUPAC gold book (https://goldbook.iupac.org/terms/view/P04479).

By "polypeptides" is meant peptides containing ten or more amino acid residues. Polypeptides are specific type of peptides.

In the instant specification and unless otherwise stated "peptides" and "polypeptides" have a molecular weight of less than or equal to 10 000.

By "proteins" is meant naturally occurring or synthetic polypeptides having molecular weight greater than about 10 000. This definition comes from IUPAC gold book.

In an embodiment, the composition comprises at least 300 mg/g of PE relative to the total weight of the composition.

In an embodiment, the composition comprises at least 400 mg/g of PE relative to the total weight of the composition.

In an embodiment, the composition comprises at least 500 mg/g of PE relative to the total weight of the composition.

In an embodiment, the composition comprises at least 600 mg/g of PE relative to the total weight of the composition.

In an embodiment, the composition comprises at least 700 mg/g of PE relative to the total weight of the composition.

In an embodiment, the composition comprises at least 800 mg/g of PE relative to the total weight of the composition.

In an embodiment, the composition comprises at least 900 mg/g of PE relative to the total weight of the composition.

In an embodiment, the composition comprises at most 980 mg/g of PE relative to the total weight of the composition.

In an embodiment, the composition comprises at most 950 mg/g of PE relative to the total weight of the composition.

In an embodiment, the composition comprises at most 900 mg/g of PE relative to the total weight of the composition.

In an embodiment, the composition comprises at most 800 mg/g of PE relative to the total weight of the composition.

In an embodiment, the composition according to the invention comprises at least one further permeation enhancer in addition to PE, this further permeation enhancer is abbreviated fPE.

In an embodiment the further permeation enhancer is chosen from the group consisting of caprate, in particular sodium caprate, caprylate, in particular sodium caprylate, bile salts, glycocholate, ursodeoxycholic acid, glycochenodeoxycholate, glycodeoxycholate and their pharmaceutically acceptable salts, and mixtures thereof.

In an embodiment, the composition comprises a bile salt. According to an embodiment the composition comprises only one bile salt.

According to an embodiment the composition comprises a mixture of bile salts.

In an embodiment the bile salt is chosen from the group consisting of glycocholate, ursodeoxycholic acid, glycochenodeoxycholate, glycodeoxycholate and their pharmaceutically acceptable salts, and mixtures thereof.

According to an embodiment the bile salt is glycocholate, in particular sodium glycocholate.

In an embodiment the further permeation enhancer is chosen from the group consisting of caprate, in particular sodium caprate, caprylate, in particular sodium caprylate.

In an embodiment the further permeation enhancer is chosen from the group consisting of caprate, in particular sodium caprate.

In an embodiment, the composition comprises at least 300 mg/g of permeation enhancers, PE + fPE, relative to the total weight of the composition.

In an embodiment, the composition comprises at least 400 mg/g of permeation enhancers relative to the total weight of the composition.

In an embodiment, the composition comprises at least 500 mg/g of permeation enhancers to the total weight of the composition.

In an embodiment, the composition comprises at least 600 mg/g of permeation enhancers relative to the total weight of the composition.

In an embodiment, the composition comprises at least 700 mg/g of permeation enhancers relative to the total weight of the composition.

In an embodiment, the composition comprises at least 800 mg/g of permeation enhancers relative to the total weight of the composition.

In an embodiment, the composition comprises at least 900 mg/g of permeation enhancers relative to the total weight of the composition.

In an embodiment, the composition comprises at most 990 mg/g of permeation enhancers relative to the total weight of the composition.

In an embodiment, the composition comprises at most 980 mg/g of permeation enhancers relative to the total weight of the composition.

In an embodiment, the composition comprises at most 950 mg/g of permeation enhancers relative to the total weight of the composition.

In an embodiment, the composition comprises at most 900 mg/g of permeation enhancers relative to the total weight of the composition.

In an embodiment, the composition comprises at most 800 mg/g of permeation enhancers relative to the total weight of the composition.

In an embodiment, the composition comprises a protease inhibitor.

In an embodiment, the composition comprises a protease inhibitor chosen from the group consisting of serine protease inhibitor, Metalloproteases inhibitors, and mixtures thereof.

In an embodiment, the composition comprises a serine protease inhibitor.

In an embodiment, the composition comprises a serine protease inhibitor chosen from the group consisting of proteines, peptides, serpins, chemicals and mixtures thereof as listed in the following paragraphs.

In an embodiment, the composition comprises a serine protease inhibitor which is a proteine or a peptide chosen from the group consisting of Aprotinin, Bacitracin, Lima bean trypsin inhibitor, Ovomucoid, Soybean trypsin inhibitor (SBTI), KTI (Kunitz Trypsine Inhibitor), BBI (Bowman-Birk Inhibitor), and mixtures thereof.

In an embodiment, the composition comprises a serine protease inhibitor which is a serpin chosen from the group consisting of Alpha-1-antitrypsin, Alpha 1-antichymotrypsin, alpha 2-macroglobulin, Antithrombin, Centerin, Kallistatin, Neuroserpin, Pancpin, Plasminogen activator inhibitor-2, Protein C inhibitor, Secretory leucocyte protease inhibitor, Squamous cell carcinoma antigen-1, Squamous cell carcinoma antigen-2, Ulinastatin, Vaspin, and mixtures thereof.

In an embodiment, the composition comprises a serine protease inhibitor which is a chemical chosen form the group consisting of AEBSF-hydrochloride (4-(2-aminoethyl)benzenesulfonyl fluoride), Aminobenzamidine dihydrochloride, epsilon-aminocaproic acid, APMSF-hydrochloride, benzamidine-hydrochloride, camostat mesylate, chymostatin, FK448, leupeptin, PEFABLOC SC, PMSF (Phenylmethylsulfonyl fluoride), TLCK (Na-Tosyl-Lys-chloromethylketone), TPCK (6-(1-tosylamido-2-phenyl) ethyl chloromethyl ketone), and mixtures thereof.

In an embodiment, the composition comprises a serine protease inhibitor chosen from the group consisting of SBTI, KTI, BBI, aprotinin, ovomucoid, and mixtures thereof.

In an embodiment, the composition comprises a serine protease inhibitor chosen from the group consisting of SBTI, KTI, BBI and mixtures thereof, particularly SBTI.

According to an embodiment the composition comprises only one serine protease inhibitor.

According to an embodiment, the composition comprises SBTI.

According to an embodiment, the composition comprises KTI.

According to an embodiment, the composition comprises BBI.

According to an embodiment the composition comprises a mixture of serine protease inhibitor.

According to an embodiment the composition comprises a mixture of serine protease inhibitor chosen from the group consisting of SBTI and aprotinin, KTI and aprotinin, BBI and aprotinin, ovomucoid and aprotinin, SBTI and ovomucoid, KTI and ovomucoid, BBI and ovomucoid.

In an embodiment, the composition comprises a metalloprotease inhibitor.

In an embodiment the composition comprises as metalloproteases inhibitor CPB (Carboxypeptidase) Inhibitor from potato tuber.

According to an embodiment the composition comprises only one metalloprotease inhibitor.

According to an embodiment the composition comprises a mixture of metalloprotease inhibitors.

According to an embodiment the composition comprises a mixture of serine protease inhibitor and metalloprotease inhibitor.

According to an embodiment the composition comprises a mixture of serine protease inhibitor and metalloprotease inhibitor is chosen from the group consisting of SBTI and CPB inhibitor, BBI and CPB inhibitor, KTI and CPB inhibitor aprotinin and CPB inhibitor, ovomucoid and CPB inhibitor.

According to an embodiment the composition comprises a mixture of serine protease inhibitor and metalloprotease inhibitor.

According to an embodiment the composition comprises the mixture of serine protease inhibitor and metalloprotease inhibitor is chosen from the group consisting of SBTI and sodium glycocholate, aprotinin and sodium glycocholate, ovomucoid and sodium glycocholate.

According to an embodiment the composition comprises from 10 to 500 mg of protease inhibitor.

According to an embodiment the composition comprises from 10 to 200 mg of protease inhibitor.

According to an embodiment the composition comprises from 10 to 150 mg of protease inhibitor.

According to an embodiment the composition comprises from 10 to 100 mg of protease inhibitor.

According to an embodiment the composition comprises from 20 to 600 mg/g of protease inhibitor.

According to an embodiment the composition comprises from 20 to 400 mg/g of protease inhibitor.

According to an embodiment the composition comprises from 20 to 250 mg of protease inhibitor.

According to an embodiment the composition comprises from 20 to 150 mg of protease inhibitor.

In an embodiment the composition comprises SBTI, KTI, BBI and their mixtures, and in particular SBTI, from 100 to 400 mg/g.

In an embodiment the composition comprises SBTI, KTI, BBI and their mixtures, and in particular SBTI, from 120 to 300 mg/g.

In an embodiment the composition comprises SBTI, KTI, BBI and their mixtures, and in particular SBTI, from 140 to 250 mg/g.

In an embodiment the composition comprises SBTI, KTI, BBI and their mixtures, and in particular SBTI, from 150 to 200 mg/g.

In an embodiment, the composition comprises a chelator of divalent cations.

In an embodiment, this chelator is a physiologically acceptable compound having a high affinity for at least one of calcium, magnesium, and manganese ions.

In another embodiment, the chelator is selected from the group consisting of ethylenediamine tetracetic acid (EDTA) or a salt thereof (for example disodium EDTA and calcium disodium EDTA); EGTA (ethylene glycol tetraacetic acid) or a salt thereof; diethylene triamine pentaacetic acid (DTPA) or a salt thereof; BAPTA (1,2-bis(o-aminophenoxy)ethane-N,N,N',N'-tetraacetic acid) or a salt thereof; and mixtures thereof.

In another embodiment, only one of the above-listed chelators is present in the composition.

In another embodiment, the chelator is EDTA.

In an embodiment the composition comprises 1 to 50 % w/w of chelator of divalent cations, in particular EDTA.

In an embodiment the composition comprises 2 to 30 % w/w of chelator of divalent cations, in particular EDTA.

In an embodiment the composition comprises 5 to 25 % w/w of chelator of divalent cations, in particular EDTA.

In an embodiment, the peptide or protein is a therapeutic peptide or protein.

In an embodiment, the peptide or protein is long acting.

By « long acting » is meant having a half life in plasma of at least 1 day.

In an embodiment long acting peptide or protein have a half life in plasma of at least 2 days.

In an embodiment long acting peptide or protein have a half life in plasma of at least 3 days.

In an embodiment long acting peptide or protein have a half life in plasma of at least 5 days.

In an embodiment long acting peptide or protein have a half life in plasma of at least 7 days.

In an embodiment long acting peptide or protein have a half life in plasma of at least 10 days.

In an embodiment long acting peptide or protein have a half life in plasma of at least 15 days.

In an embodiment, the peptide or protein is short acting.

By « short acting » is meant having a half life in plasma of less than 1 day.

In an embodiment short acting peptide or protein have a half life in plasma of at most 18 hours.

In an embodiment short acting peptide or protein have a half life in plasma of at most 12 hours.

In an embodiment short acting peptide or protein have a half life in plasma of at most 6 hours.

In an embodiment short acting peptide or protein have a half life in plasma of at most 4 hours.

In an embodiment short acting peptide or protein have a half life in plasma of at most 2 hours.

In an embodiment, the peptide or protein has a solubility in water at 25°C or more of at least 10 mg/ml.

In an embodiment, the peptide or protein has a solubility in water at 25°C or more of at least 20 mg/ml.

In an embodiment, the peptide or protein has a solubility in water at 25°C or more of at least 30 mg/ml.

In an embodiment, the peptide or protein has a solubility in water at 25°C or more of at least 40 mg/ml.

In an embodiment, the peptide or protein has a solubility in water at 25°C or more of at least 50 mg/ml.

In an embodiment, the peptide or protein has a solubility in water at 25°C or more of at most 40 mg/ml.

In an embodiment, the peptide or protein has a solubility in water at 25°C or more of at most 30 mg/ml.

In an embodiment, the peptide or protein has a solubility in water at 25°C or more of at most 20 mg/ml.

In an embodiment, the peptide or protein has a solubility in water at 25°C or more of at most 10 mg/ml.

In an embodiment, the peptide or protein has a molecular weight of at least 1500 Da.

In an embodiment, the peptide or protein has a molecular weight of at least 2000 Da.

In an embodiment, the peptide or protein has a molecular weight of at least 2500 Da.

In an embodiment, the peptide or protein has a molecular weight of at least 3000 Da.

In an embodiment, the peptide or protein has a molecular weight of at least 3500 Da.

In an embodiment, the peptide or protein has a molecular weight of at least 4000 Da.

In an embodiment, the peptide or protein has a molecular weight of at most 20000 Da.

In an embodiment, the peptide or protein has a molecular weight of at most 15000 Da.

In an embodiment, the peptide or protein has a molecular weight of at most 10000 Da.

In an embodiment, the peptide or protein has a molecular weight of at most 5000 Da.

In an embodiment, the composition comprises from 0.25 to 20 wt% of peptide or protein.

In an embodiment, the composition comprises from 0.5 to 20 wt% of peptide or protein.

In an embodiment, the composition comprises from 1 to 20 wt% of peptide or protein.

In an embodiment, the composition comprises from 2 to 20 wt% of peptide or protein.

In an embodiment, the composition comprises from 5 to 20 wt% of peptide or protein.

In an embodiment, the composition comprises from 1 to 15 wt% of peptide or protein.

In an embodiment, the composition comprises from 2 to 15 wt% of peptide or protein.

In an embodiment, the composition comprises from 5 to 15 wt% of peptide or protein.

In an embodiment, the peptide or protein is chosen from the group consisting of GLP-1 RA, GLP-2 RA, insulin and insulin analogs, amylin RA, GIP RA, PYY RA, dual agonists GIP/GLP-1, dual agonists GLP-1/glucagon, ParaThyroid Hormones (PTH) and PTH analogs, InterLeukines (IL) and IL analogs, Growth Hormones (GH) and GH analogs, Insulin Growth Factors (IGF) and IGF analogs, Interferons (IFN) and IFN analogs.

In an embodiment, the peptide or protein is octreotide

In an embodiment, the peptide or protein is a GLP-1 RA chosen from the group consisting of semaglutide.

In an embodiment, the peptide or protein is a GLP-1 RA chosen from the group consisting of dulaglutide.

In an embodiment, the peptide or protein is a GLP-2 RA chosen from the group consisting of teduglutide.

In an embodiment, the peptide or protein is insulin or an insulin analog. In particular the insulin analog is chosen from the group consisting of degludec and detemir.

In an embodiment, the peptide or protein is an amylin RA or an amylin analog, in particular chosen from the group consisting of pramlintide, cagrilintide.

In an embodiment, the peptide or protein is a GIP RA chosen from the group consisting of GIP RA disclosed in WO2021021877, WO16066744 and WO2018181864.

In an embodiment, the peptide or protein is a PYY RA chosen from the group consisting of PYY (1-36), PYY (3-36) and PYY RA disclosed in WO2021023817, WO19147650 and WO2016198682.

In an embodiment, the peptide or protein is a dual agonist GIP/GLP-1 chosen from the group consisting of dual agonist GIP/GLP-1 disclosed in WO2016111971, and in particular Tirzepatide.

In an embodiment, the peptide or protein is a dual agonists GLP-1/glucagon.

In an embodiment, the peptide or protein is an analog of oxyntomodulin.

In an embodiment, the peptide or protein is a ParaThyroid Hormone (PTH) or a PTH analog chosen from the group consisting of human PTH and PTH analogs.

In an embodiment, the peptide or protein is an InterLeukines (IL) or IL analog chosen from the group consisting of IL-11 and analogs.

In an embodiment, the peptide or protein is a Growth Hormone (GH) or a GH analog chosen from the group consisting of Human Growth Hormone and analogs, in particular human growth hormone bearing an acylated graft.

In an embodiment, the peptide or protein is an Insulin Growth Factor (IGF) or an IGF analog chosen from the group consisting of IGF-1.

In an embodiment, the peptide or protein is an Interferon (IFN) or an IFN analog chosen from the group consisting of INF beta-la, INF beta-lb and INF gamma.

In an embodiment, the peptide or protein is a peptide or protein comprising modifications in the form of a covalent modification such as a side chain attached to one or more amino acids of the hydrophylic peptide or protein.

In an embodiment, the peptide or protein is a peptide or protein comprising modifications in the form of an attachment of amides, carbohydrates, alkyl groups, acyl groups, esters, PEGylations and the like.

In an embodiment, the peptide or protein is a peptide or protein comprising modifications in the form of an attachment at least one acyl group, said acyl group comprising at least 10 carbon atoms.

In an embodiment, the acyl moiety is -OEG-OEG-gamma-L-Glu-octadecanedioyl, wherein OEG is -COCH₂O(CH₂)₂0O(CH₂)₂NH-

In an embodiment, the peptide or protein is a peptide or protein comprising modifications in the form of an attachment at least one PEGylation.

In an embodiment the composition does not comprise an acylated aminoacid AC-aa, or a salt thereof, wherein the acyl group AC comprises from 4 to 9 carbon atoms, in particular 4 to 8 carbon atoms, as these compounds are protease inhibitors.

In an embodiment the composition does not comprise an AC-aa such as disclosed in the european patent application havind the filing number EP21208347.1.

In an embodiment, the weight ratio PE / peptide or protein is going from 1:1 to 200:1.

In an embodiment, the weight ratio PE / peptide or protein is going from 1:1 to 100:1.

In an embodiment, the weight ratio PE / peptide or protein is going from 1:1 to 50:1.

In an embodiment, the weight ratio PE / peptide or protein is going from 2:1 to 40:1.

In an embodiment, the weight ratio PE / peptide or protein is going from 4:1 to 30:1.

In an embodiment, the weight ratio PE / peptide or protein is going from 6:1 to 20:1.

In an embodiment, the composition comprises less than 10 % w/w of water.

In an embodiment, the composition comprises less than 5 % w/w of water.

In an embodiment, the composition comprises less than 2 % w/w of water.

In an embodiment, the composition comprises less than 1 % w/w of water.

In an embodiment, the solid composition comprises a mixture of particles comprising the PE, or PE + fPE, and particles comprising the peptide or protein. In particular the PE and the peptide or protein are present in the composition in the ratio and percentages as disclosed above.

In an embodiment, the solid composition consists of a mixture of particles comprising PE, or PE + fPE, and of particles comprising the peptide or protein. In particular the PE and the peptide or protein are present in the composition in the ratio and percentages as disclosed above.

In an embodiment, the solid composition comprises particles wherein the PE, or PE + fPE, and the peptide or protein are mixed. In particular in the ratio and percentages as disclosed above.

In an embodiment, the solid composition consists of particles wherein the PE, or PE + fPE, and the peptide or protein are mixed. In particular in the ratio and percentages as disclosed above.

In an embodiment, the solid composition consists of particles wherein the PE, or PE + fPE, and the peptide or protein are mixed. In particular in the ratio and percentages as disclosed above.

The particles comprising at the same time peptide or protein and PE, or PE + fPE, may be obtained via lyophilization, freeze drying, spray drying, wet granulation or dry granulation.

In an embodiment, the particles are chosen in a group consisting of microgranules (5-0.5mm, 0.5 mm excluded), microparticules (0.5mm-1µm, 1 µm excluded) or nanoparticles (1000-1nm) and mixtures thereof.

In an embodiment, the particles are microgranules (number-averaged mean diameter 5.0-0.5mm), for instance measured by laser granulometry.

In an embodiment, the particles are microparticules (number-averaged mean diameter 0.5mm-1µm), for instance measured by light obscuration.

In an embodiment, the particles are nanoparticles (number-averaged mean diameter 1000-1nm), for instance measured by electron microscopy.

Among manufacturing methods to reach these specific sizes the following methods can be cited wet granulation, dry granulation, spray-drying, milling, nanoprecipitation.

In an embodiment these particles are free from each other.

In another embodiment, these particles are held together.

In an embodiment, the composition comprises excipients chosen from the list consisting of lubricants, surfactants, pH modifiers, disintegrants, binders, fillers, glidants, diluents, polymers for sustained or delayed release (e.g. Eudragit polymers manufactured by Evonik) and preservatives.

In an embodiment, the composition comprises only excipients chosen from the list consisting of lubricants, surfactants, pH modifiers, disintegrants, binders, fillers, glidants, diluents and preservatives.

In an embodiment, the composition comprises only excipients chosen from the list consisting of lubricants, pH modifiers.

In an embodiment, the composition comprises at most 20 % w/w of excipients, in particular of excipients such as listed above, more particularly components belonging to the lists in the 3 paragraphs above.

By "excipients" is meant components of the composition which are not permeation enhancers and protease inhibitors.

In an embodiment, the composition comprises at most 15 % w/w of excipients, in particular of excipients such as listed above.

In an embodiment, the total amount of binder, filler and glidant is at most 10 % w/w of the composition.

In an embodiment, the composition comprises at least one pH modifier.

In an embodiment the pH modifier can be chosen from the group consisting of sodium carbonate, which formula is Na₂CO₃, phosphates, citrates, citric acid, tartarate and tartaric acid.

Citrates can be monosodium citrate, disodium citrate and/or trisodium citrate. In particular it can be trisodium citrate.

Tartarate can be monosodium and/or disodium tartarate. In particular this is monosodium tartarate.

In an embodiment the pH modifier is sodium carbonate, which formula is Na2C03.

In an embodiment the composition comprises at least 1 % w/w of pH modifier.

In an embodiment the composition comprises at least 2 % w/w of pH modifier.

In an embodiment the composition comprises at least 5 % w/w of pH modifier.

In an embodiment the composition comprises at most 15 % w/w of pH modifier.

In an embodiment the composition comprises at most 10 % w/w of pH modifier.

In an embodiment the composition comprises at most 8 % w/w of pH modifier.

In an embodiment the composition comprises at least one lubricant.

In an embodiment the lubricant is chosen from the group consisting of magnesium stearate or glyceryl dibehenate.

In an embodiment the lubricant is magnesium stearate.

In an embodiment the lubricant is glyceryl dibehenate.

In an embodiment the composition comprises more than or is equal to 0.1 % w/w of lubricant.

In an embodiment the composition comprises more than or is equal to 0.2 % w/w of lubricant.

In an embodiment the composition comprises more than or is equal to 0.5 % w/w of lubricant.

In an embodiment the composition comprises less than 5 % w/w of lubricant.

In an embodiment the composition comprises less than 3 % w/w of lubricant.

In an embodiment the composition comprises less than 2.5 % w/w of lubricant.

In an embodiment the composition comprises less than 2 % w/w of lubricant.

In an embodiment the composition comprises less than 1 % w/w of lubricant.

In an embodiment the composition comprises between 0.25 and 2.5 % w/w of lubricant.

In an embodiment, the composition is in the form of a unitary solid dosage form, such as capsules, tablets, dragees, pills, lozenges, powders, and granules.

In an embodiment, the composition is in the form of a unitary solid dosage form, such as capsules, tablets, dragees, pills, lozenges, powders, and granules, said unitary dosage form comprising at least 50 mg of PE.

In an embodiment, the unitary solid dosage form is under the form of a capsule.

In an embodiment, the unitary solid dosage form is under the form of a hard capsule.

In an embodiment, the unitary solid dosage form is under the form of a soft capsule.

The capsules may contain powders, granules, crushed tablets that contains the peptide or a protein and one or more inert ingredients. Capsules can be formulated with delayed release characteristics.

In an embodiment the composition is encapsulated or the like to allow the composition which is swallowed to be in contact with the gastro intestinal system.

In an embodiment the encapsulation allows the composition to be delivered in the stomach.

Thus the composition may be in the form of a film coated tablets with a thin layer of water soluble material that dissolves rapidly in the stomach.

In another embodiment the encapsulation allows the composition to be delivered in the intestines.

Thus the composition may be encapsulated with an enteric coating. This enteric coating may be in the form of a polymer coating or of a polymer capsule that controls disintegration and release of the composition.

The enteric coating may be soft technology. In an embodiment it is soft capsule technnology.

The enteric coating may be hard technology. In an embodiment it is hard capsule technnology.

The term "enteric coating" means a coating that controls disintegration and release of the oral dosage form.

The site of disintegration and release of the solid dosage form may be designed depending on the pH of the targeted area, where absorption of the peptide or protein is desired, thus also includes acid resistant protective coatings.

In an embodiment, the solid oral dosage form allow a release at pH from 4.5 and above.

In an embodiment, the solid oral dosage form allow a release at pH from 4.75 and above.

In an embodiment, the solid oral dosage form allow a release at pH from 5.0 and above.

In an embodiment, the solid oral dosage form allow a release at pH from 5.25 and above.

In an embodiment, the solid oral dosage form allow a release at a pH from 5.5 and above.

In an embodiment, the solid oral dosage form allow a release at a pH from 5.75 and above.

In an embodiment, the solid oral dosage form allow a release at a pH from 6.0 and above.

The site of disintegration and release of the solid oral dosage form may be designed using delayed-release technologies, such as polymers with a controlled solubilization rate in aqueous medium, for instance Eudragit RL or RS polymers.

In an embodiment the Solid Oral Dosage Form according to the invention at pH 4.5 is considered as an Immediate-Release Solid Oral Dosage Form.

In an embodiment the Solid Oral Dosage Form according to the invention at pH 5.0 is considered as an Immediate-Release Solid Oral Dosage Form.

In an embodiment the Solid Oral Dosage Form according to the invention at pH 5.5 is considered as an Immediate-Release Solid Oral Dosage Form.

In an embodiment the Solid Oral Dosage Form according to the invention at pH 6.0 is considered as an Immediate-Release Solid Oral Dosage Form.

To be considered as an Immediate-Release Solid Oral Dosage Form means that the solid dosage oral form complies with the conditions disclosed in "Dissolution Testing and Acceptance Criteria for Immediate-Release Solid Oral Dosage Form Drug Products Containing High Solubility Drug Substances Guidance for Industry" - August 2018(Additional copies are available from: Office of Communications, Division of Drug Information Center for Drug Evaluation and Research Food and Drug Administration 10001 New Hampshire Ave., Hillandale Bldg., 4th Floor Silver Spring, MD 20993-0002 Phone: 855-543-3784 or 301-796-3400; Fax: 301-431-6353 Email: druginfo@fda.hhs.gov http://www.fda.gov/Drugs/GuidanceComplianceRegulatoryInformation/Guidances/def ault.htm).

In an embodiment the solid dosage oral form comprises from 50 to 250 mg of SBTI, KTI, BBI and their mixtures, and in particular of SBTI.

In an embodiment the solid dosage oral form comprises from 60 to 200 mg of SBTI, KTI, BBI and their mixtures, and in particular of SBTI.

In an embodiment the solid dosage oral form comprises from 70 to 150 mg of SBTI, KTI, BBI and their mixtures, and in particular of SBTI.

In an embodiment, the composition is for use as a medicament.

In an embodiment the composition is for preventing or treating obesity, Diabetes Type 1, Diabetes Type 2, NASH, thrombocytopaenia, Short Bowel Syndrom (SBS), adult GH deficiency, GH disorders, Short stature syndrome, Turner's syndrome, Achondroplasia, Prader-Willi syndrome, Short stature in children, osteoporosis and hypoparathyroidism, Multiple sclerosis, Bone disorders.

In an embodiment the composition is for preventing or treating obesity.

In an embodiment the composition is for preventing or treating Diabetes Type 1.

In an embodiment the composition is for preventing or treating Diabetes Type 2.

In an embodiment the composition is for preventing or treating NASH.

In an embodiment the composition is for preventing or treating thrombocytopaenia.

In an embodiment the composition is for preventing or treating Short Bowel Syndrom (SBS).

In an embodiment the composition is for preventing or treating adult GH deficiency, GH disorders, Short stature syndrome, Turner's syndrome, Achondroplasia, Prader-Willi syndrome or Short stature in children.

In an embodiment the composition is for preventing or treating osteoporosis.

In an embodiment the composition is for preventing or treating hypoparathyroidism.

In an embodiment the composition is for preventing or treating Multiple sclerosis.

In an embodiment the composition is for preventing or treating Bone disorders.

According to an embodiment, the composition comprises the PE, in particular SNAC or KNAC, a peptide or a protein, and a lubricant.

According to an embodiment, the composition comprises the PE, in particular SNAC or KNAC, a peptide or a protein, and a lubricant which is magnesium stearate.

According to an embodiment, the composition comprises the PE, in particular SNAC or KNAC, a peptide or a protein, and a lubricant which is glyceryl dibehenate.

According to an embodiment, the composition comprises the PE, in particular SNAC or KNAC, a peptide or a protein, a protease inhibitor, and a lubricant.

According to an embodiment, the composition comprises the PE, in particular SNAC or KNAC, a peptide or a protein and a lubricant which is chosen from the group consisting of magnesium stearate or glyceryl dibehenate.

According to an embodiment, the composition comprises the PE, in particular SNAC or KNAC, a peptide or a protein and a further permeation enhancer, fPE.

According to an embodiment, the composition comprises the PE, in particular SNAC or KNAC, a peptide or a protein and a further permeation enhancer which is chosen from the group consisting of caprate, in particular sodium caprate, caprylate, in particular sodium caprylate, and bile salts.

According to an embodiment, the composition comprises the PE, in particular SNAC or KNAC, a peptide or a protein and a pH modifier.

According to an embodiment, the composition comprises the PE, in particular SNAC or KNAC, a peptide or a protein and a pH modifier which is chosen from the group consisting of sodium carbonate, which formula is Na₂CO₃, phosphates, citrates, citric acid, tartarate and tartaric acid.

According to an embodiment, the composition comprises the PE, in particular SNAC or KNAC, a peptide or a protein and a pH modifier which is sodium carbonate.

According to an embodiment, the composition comprises the PE, in particular SNAC or KNAC, a peptide or a protein and a protease inhibitor.

According to an embodiment, the composition comprises the PE, in particular SNAC or KNAC, with an AC group comprising 8 carbon atoms, a peptide or a protein, a pH modifier and a protease inhibitor.

According to an embodiment, the composition comprises the PE, in particular SNAC or KNAC, a peptide or a protein, a further permeation enhancer and a protease inhibitor.

According to an embodiment, the composition comprises the PE, in particular SNAC or KNAC, a peptide or a protein, a lubricant and a protease inhibitor.

According to an embodiment, the composition comprises the PE, in particular SNAC or KNAC, a peptide or a protein, a pH modifier, a further permeation enhancer and a protease inhibitor.

According to an embodiment, the composition comprises the PE, in particular SNAC or KNAC, a peptide or a protein, a lubricant and a pH modifier.

According to an embodiment, the composition comprises the PE, in particular SNAC or KNAC, a peptide or a protein, a lubricant which is magnesium stearate and a pH modifier.

According to an embodiment, the composition comprises the PE, in particular SNAC or KNAC, a peptide or a protein, a lubricant which is magnesium stearate and a pH modifier which is sodium carbonate.

According to an embodiment, the composition comprises the PE, in particular SNAC or KNAC, a peptide or a protein, a lubricant which is glyceryl dibehenate and a pH modifier.

According to an embodiment, the composition the PE, in particular SNAC or KNAC, a peptide or a protein, and a lubricant which is glyceryl dibehenate and a pH modifier which is sodium carbonate.

According to an embodiment, the composition comprises the PE, in particular SNAC or KNAC, a peptide or a protein, a lubricant and a disintegrant.

According to an embodiment, the composition comprises the PE, in particular SNAC or KNAC, a peptide or a protein, a lubricant which is magnesium stearate and a disintegrant.

According to an embodiment, the composition comprises the PE, in particular SNAC or KNAC, a peptide or a protein, a lubricant which is glyceryl dibehenate and a disintegrant.

According to an embodiment, the composition comprises the PE, in particular SNAC or KNAC, a peptide or a protein, a lubricant, a pH modifier and a disintegrant.

According to an embodiment, the composition the PE, in particular SNAC or KNAC, a peptide or a protein, a lubricant which is magnesium stearate,a pH modifier and a disintegrant.

According to an embodiment, the composition comprises the PE, in particular SNAC or KNAC, a peptide or a protein, a lubricant which is magnesium stearate, a pH modifier which is sodium carbonate and a disintegrant.

According to an embodiment, the composition comprises the PE, in particular SNAC or KNAC, a peptide or a protein, a lubricant which is glyceryl dibehenate, a pH modifier and a disintegrant.

According to an embodiment, the composition comprises the PE, in particular SNAC or KNAC, a peptide or a protein, and a lubricant which is glyceryl dibehenate, a pH modifier which is sodium carbonate and a disintegrant.

In an embodiment, the composition comprises a PE, in particular SNAC or KNAC, and a protein or a peptide.

### PE / SNAC OK

In an embodiment, the composition comprises a PE, in particular SNAC or KNAC, a protein or a peptide, and a further permeation enhancer.

### Inhib Prot / SBTI BBI

In an embodiment, the composition comprises a PE, in particular SNAC or KNAC, a protein or a peptide, and a protease inhibitor, in particular chosen from the group consisting of SBTI, BBI and KTI.

### EDTA

In an embodiment, the composition comprises a PE, in particular SNAC or KNAC, a protein or a peptide, and a chelator of divalent cation, in particular EDTA.

### pH Mod Na2CO3

In an embodiment, the composition comprises a PE, in particular SNAC or KNAC, a protein or a peptide, and a pH modifier, in particular carbonate, more particularly Na2C03.

### Disintegrant

In an embodiment, the composition comprises a PE, in particular SNAC or KNAC, a protein or a peptide, and a disintegrant, in particular croscarmellose.

### PE / SNAC + Inhib Prot / SBTI BBI

In an embodiment, the composition comprises a PE, in particular SNAC or KNAC, a protein or a peptide, a further permeation enhancer and a protease inhibitor, in particular chosen from the group consisting of SBTI, BBI and KTI.

### PE / SNAC + EDTA

In an embodiment, the composition comprises a PE, in particular SNAC or KNAC, a protein or a peptide, a further permeation enhancer and a chelator of divalent cation, in particular EDTA.

### PE / SNAC + pH Mod Na2CO3

In an embodiment, the composition comprises a PE, in particular SNAC or KNAC, a protein or a peptide, a further permeation enhancer and a pH modifier, in particular carbonate, more particularly Na2C03.

### PE / SNAC + Disintegrant

In an embodiment, the composition comprises a PE, in particular SNAC or KNAC, a protein or a peptide, a further permeation enhancer, and a disintegrant, in particular croscarmellose.

### Inhib Prot / SBTI BBI + EDTA

In an embodiment, the composition comprises a PE, in particular SNAC or KNAC, a protein or a peptide, a protease inhibitor, in particular chosen from the group consisting of SBTI, BBI and KTI, and a chelator of divalent cation, in particular EDTA.

### Inhib Prot / SBTI BBI + pH Mod Na2CO3

In an embodiment, the composition comprises a PE, in particular SNAC or KNAC, a protein or a peptide, a protease inhibitor, in particular chosen from the group consisting of SBTI, BBI and KTI, and a pH modifier, in particular carbonate, more particularly Na2C03.

### Inhib Prot / SBTI BBI + Disintegrant

In an embodiment, the composition comprises a PE, in particular SNAC or KNAC, a protein or a peptide, a protease inhibitor, in particular chosen from the group consisting of SBTI, BBI and KTI, and a disintegrant, in particular croscarmellose.

### PE / SNAC + Inhib Prot / SBTI BBI + EDTA

In an embodiment, the composition comprises a PE, in particular SNAC or KNAC, a protein or a peptide, a further permeation enhancer, a protease inhibitor, in particular chosen from the group consisting of SBTI, BBI and KTI, and a chelator of divalent cation, in particular EDTA.

### PE / SNAC + Inhib Prot / SBTI BBI + pH Mod Na2CO3

In an embodiment, the composition comprises a PE, in particular SNAC or KNAC, a protein or a peptide, a further permeation enhancer, a protease inhibitor, in particular chosen from the group consisting of SBTI, BBI and KTI, and a pH modifier, in particular carbonate, more particularly Na2C03.

### PE / SNAC + Inhib Prot / SBTI BBI + Disintegrant

In an embodiment, the composition comprises a PE, in particular SNAC, a protein or a peptide, a further permeation enhancer, a protease inhibitor, in particular chosen from the group consisting of SBTI, BBI and KTI, and a disintegrant, in particular croscarmellose.

### PE / SNAC + EDTA + pH Mod Na2CO3

In an embodiment, the composition comprises a PE, in particular SNAC or KNAC, a protein or a peptide, a further permeation enhancer, a chelator of divalent cation, in particular EDTA, and a pH modifier, in particular carbonate, more particularly Na2C03.

### PE / SNAC + EDTA + Disintegrant

In an embodiment, the composition comprises a PE, in particular SNAC or KNAC, a protein or a peptide, a further permeation enhancer, a chelator of divalent cation, in particular EDTA, and a disintegrant, in particular croscarmellose.

### Inhib Prot / SBTI BBI + EDTA + pH Mod Na2CO3

In an embodiment, the composition comprises a PE, in particular SNAC or KNAC, a protein or a peptide, a protease inhibitor, in particular chosen from the group consisting of SBTI, BBI and KTI, a chelator of divalent cation, in particular EDTA, and a pH modifier, in particular carbonate, more particularly Na2C03.

### Inhib Prot / SBTI BBI + EDTA + Disintegrant

In an embodiment, the composition comprises a PE, in particular SNAC or KNAC, a protein or a peptide, a protease inhibitor, in particular chosen from the group consisting of SBTI, BBI and KTI, a chelator of divalent cation, in particular EDTA, and a disintegrant, in particular croscarmellose.

### Inhib Prot / SBTI BBI + pH Mod Na2CO3 + Disintegrant

In an embodiment, the composition comprises a PE, in particular SNAC or KNAC, a protein or a peptide, a protease inhibitor, in particular chosen from the group consisting of SBTI, BBI and KTI, a pH modifier, in particular carbonate, more particularly Na2CO3, and a disintegrant, in particular croscarmellose.

### PE / SNAC + Inhib Prot / SBTI BBI + EDTA + pH Mod Na2CO3

In an embodiment, the composition comprises a PE, in particular SNAC or KNAC, a protein or a peptide, a further permeation enhancer, a protease inhibitor, in particular chosen from the group consisting of SBTI, BBI and KTI, a chelator of divalent cation, in particular EDTA, and a pH modifier, in particular carbonate, more particularly Na2C03.

### PE / SNAC + Inhib Prot / SBTI BBI + EDTA + Disintegrant

In an embodiment, the composition comprises a PE, in particular SNAC or KNAC, a protein or a peptide, a further permeation enhancer, a protease inhibitor, in particular chosen from the group consisting of SBTI, BBI and KTI, a chelator of divalent cation, in particular EDTA, and a disintegrant, in particular croscarmellose.

### PE / SNAC + I nhib Prot + pH Mod Na2CO3 + Disintegrant

In an embodiment, the composition comprises a PE, in particular SNAC or KNAC, a protein or a peptide, a further permeation enhancer, a protease inhibitor, in particular chosen from the group consisting of SBTI, BBI and KTI, a pH modifier, in particular carbonate, more particularly Na2CO3, and a disintegrant, in particular croscarmellose.

### PE / SNAC + EDTA + pH Mod Na2CO3 + Disintegrant

In an embodiment, the composition comprises a PE, in particular SNAC or KNAC, a protein or a peptide, a further permeation enhancer, a chelator of divalent cation, in particular EDTA, a pH modifier, in particular carbonate, more particularly Na2CO3, and a disintegrant, in particular croscarmellose.

### Inhib Prot / SBTI BBI + EDTA + pH Mod Na2CO3 + Disintegrant

In an embodiment, the composition comprises a PE, in particular SNAC or KNAC, a protein or a peptide, a protease inhibitor, in particular chosen from the group consisting of SBTI, BBI and KTI, a chelator of divalent cation, in particular EDTA, a pH modifier, in particular carbonate, more particularly Na2CO3, and a disintegrant, in particular croscarmellose.

### PE / SNAC + Inhib Prot / SBTI BBI + EDTA + pH Mod Na2CO3+

### Disintegrant

In an embodiment, the composition comprises a PE, in particular SNAC or KNAC, a protein or a peptide, a further permeation enhancer, a protease inhibitor, in particular chosen from the group consisting of SBTI, BBI and KTI, a chelator of divalent cation, in particular EDTA, a pH modifier, in particular carbonate, more particularly Na2CO3, and a disintegrant, in particular croscarmellose.

### EXAMPLES

### Part A - Preparation of compositions

### A.1. Solid compositions

### Process A-1

The solid compositions were prepared by first mixing together the PE with the peptide or a protein in appropriate proportions, as well as other excipients in aqueous solution. Total solute concentration is in the range 20-500mg/g of solution. This solution is then freeze-dried to yield a homogeneous solid lyophilisate. A mortar and pestle is then used to crush this solid into a powder suitable for further processing. The resulting blend was then introduced manually in Enteric VCaps, size 00 (Capsugel).

Capsules were then sealed using the following protocol: 10µL of a 50/50 vol/vol mixture of ethanol and water were introduced between the lower part and the upper lid of the capsule using a syringe. The sealing solution was then dried under hot air (45°C) for one minute, leading to an impermeable seal between both parts of the capsule.

### Example A.1.1 : Preparation of a SNAC and semaglutide solid composition.

Composition A.1.1 is prepared according to process A.2 using 14mg of semaglutide, 400mg of SNAC, 43mg of sodium carbonate and 20mg of SBTI.

## Claims

1. Solid composition comprising a peptide or a protein and a permeation enhancer abbreviated PE, chosen from the group consisting of NAC (8-N-(2-hydroxybenzoyl)aminocaprylate), NAD (10-N-(2-hydroxybenzoyl)aminodecanoic acid), 5-CNAC (8-N-(5-chlorosalicyloyl)aminocaprylic acid), 4-MOAC (8-N-(2-hydroxy-4-methoxybenzoyl)aminocaprylic acid), 4-CNAB (4-N-(2-hydroxy-4-chlorobenzoyl)aminobutanoic acid) and salts thereof.

2. Composition according to Claim 1, **characterized in that** the composition is an oral composition.

3. Solid composition according to Claim 1 or 2, **characterized in that** in particular the sodium or potassium salt of (8-N-(2- hydroxybenzoyl)aminocaprylate), respectively called SNAC and KNAC.

4. Solid composition according to any of the preceding Claims, **characterized in that** it comprises a further permeation enhancer.

5. Solid composition according to any of the preceding Claims, **characterized in that** the the composition comprises from 0.5 to 20 wt% of a peptide or protein.

6. Solid composition according to any of the preceding Claims, **characterized in that** the peptide or protein is chosen from the group consisting of GLP-1 RA, GLP-2 RA, insulin and insulin analogs, amylin RA, GIP RA, PYY RA, dual agonists GIP/glucagon, dual agonists GLP-1/glucagon, ParaThyroid Hormones (PTH) and PTH analogs, InterLeukines (IL) and IL analogs, Growth Hormones (GH) and GH analogs, Insulin Growth Factors (IGF) and IGF analogs, Interferons (IFN) and IFN analogs.

7. Solid composition according to any of the preceding Claims, **characterized in that** it further comprises a protease inhibitor.

8. Solid composition according to any of the preceding Claims, **characterized in that** it comprises a pH modifier.

9. Solid composition according to any of the preceding Claims, **characterized in that** it comprises a chelator of divalent cation.

10. Solid composition according to any of the preceding Claims, **characterized in that** it comprises a disintegrant.

11. Solid composition according to any of the preceding Claims, **characterized in that** it comprises a lubricant.

12. Solid composition according to any of the preceding Claims, for use as a medicament.

13. Solid composition according to Claim 12, for preventing or treating obesity, Diabetes Type 1, Diabetes Type 2, NASH, thrombocytopaenia, Short Bowel Syndrom (SBS), adult GH deficiency, GH disorders, Short stature syndrome, Turner's syndrome, Achondroplasia, Prader-Willi syndrome, Short stature in children, osteoporosis and hypoparathyroidism, Multiple sclerosis, Bone disorders.

14. Solid oral dosage form **characterized in that** it comprises a composition according to any on of Claims 1 to 13
